# EUROPÄISCHE PATENTANMELDUNG

(11) **EP 3 530 224 A1**
(43) Veröffentlichungstag der Anmeldung: **28.08.2019**
(21) Anmeldenummer: 18158329.5
(22) Anmeldetag: 23.02.2018
(51) Int. Cl.: A61B 17/80

(54) **HANDGELENKSARTHRODESEPLATTE**

(71) Anmelder: Medartis Holding AG, 4057 Basel (CH)
(72) Erfinder: EBI, Daniel, 4417 Ziefen (CH); KEBRLE, Radek, Horejsi Vrchlabi 543 02 (CZ); AMMANN, Marc, 4148 Pfeffingen (CH); KAINZ, Daniel, 4056 Basel (CH); SCHÄTZLE, Simon Martin, 79288 Gottenheim (DE); TRIBELHORN, Thomas, 4497 Rünenberg (CH)
(74) Vertreter: Hepp Wenger Ryffel AG

(57) **Zusammenfassung**

Handgelenksarthrodeseplatte (20) bestehend aus einem distalen (21) und einem proximalen (22) Bereich, welcher in Richtung einer Längsachse (A) an den distalen Bereich (21) anschliesst. Form und Grösse der Platte (20) ist so ausgebildet, dass der distale Bereich (21) der Platte an der Handwurzel (1) und ein proximaler Bereich (22) der Platte am Radius (3) mit Hilfe von in Plattenlöchern einsetzbaren Befestigungsmitteln, insbesondere Schrauben, befestigbar sind. Form und Grösse eines distalen Endbereichs (29), welcher eine distalste Stelle (23) der Platte (20) umfasst, sind so ausgebildet, dass der distale Endbereich (29) mit der distalsten Stelle (23)genau und nur an einem Handwurzelknochen aus der Gruppe Os trapezium (11), Os trapezoideum (10), Os capitatum (9), und Os hamatum (8) anlegbar ist, ohne dabei einen Teil der lateral benachbarten Knochen zu überdecken.

## Beschreibung

Die Erfindung betrifft Handgelenksarthrodeseplatten und Verfahren mit Handgelenksarthrodeseplatten gemäss den kennzeichnenden Teilen der unabhängigen Ansprüche.

Eine Handgelenksarthrodese (Handgelenksversteifung) wird in der Regel bei schmerzhaften Endzuständen des Handgelenks angewandt. Solche Endzustände können ihre Ursache beispielsweise in sehr alten Kahnbeinfrakturen (Kahnbeinpseudarthrosen), im Spätstadium des Mondbeintodes (Lunatummalazie), oder in einem Zusammenbruch der Handwurzelknochen in Folge eines alten Bandrisses der Handgelenksbänder haben. Insbesondere kann eine Versteifung auch bei schmerzhafter Arthrose nach in Fehlstellung verheilten Speichenbrüchen oder bei fortgeschrittener, schmerzhafter Arthrose aus innerer Ursache erfolgen. Nach einer Handgelenksarthrodese ist das Handgelenk wieder schmerzfrei, jedoch versteift.

Für eine Handgelenksarthrodese schlägt die US 5,853,413 eine Platte vor, mit welcher der Radius (Speiche), mehrere Handwurzelknochen und ein Mittelhandknochen miteinander versteift werden. Mit der Zeit werden der Radius, die Handwurzelknochen und der Mittalhandknochen miteinander verschmelzen und das Handgelenk dadurch versteift.

Mit einer Platte nach US 5,853,413 wird das Handgelenk relativ grossflächig versteift bzw. fusioniert, wodurch die Bewegungsfreiheit des Handgelenks des Patienten nach der Versteifung sehr eingeschränkt ist.

Der Anbieter Medartis schlägt im Katalog "Arthrodesen-System 2.0/ 2.3, 2.5" eine Platte vor, bei welcher mehrere Handwurzelknochen mit dem Radius versteift werden. Die Platte erstreckt sich anders als gemäss US 5,853,413 jedoch nicht bis zu einem Mittelhandknochen.

Bei der vorgeschlagenen Platte wird das Handgelenk insgesamt zwar deutlich weniger versteift als bei einer Platte nach US 5,853,413. Allerdings ist die Bewegungsfreiheit des Handgelenks nach der Versteifung immer noch relativ stark eingeschränkt.

Es ist daher eine Aufgabe der Erfindung, die Nachteile der bekannten Platten zu minimieren und insbesondere eine Platte bereitzustellen, welche ein schmerzfreies Handgelenk mit möglichst grosser Bewegungsfreiheit ermöglicht. Die Aufgabe wird durch die Merkmale der unabhängigen Ansprüche gelöst.

Es wird eine Handgelenksarthrodeseplatte bestehend aus einem distalen und einem proximalen Bereich vorgeschlagen. Der proximale Bereich schliesst in Richtung einer Längsachse an den distalen Bereich an. Form und Grösse der Platte sind so ausgebildet, dass der distale Bereich der Platte an der Handwurzel und ein proximaler Bereich der Platte am Radius mit Hilfe von in Plattenlöchern einsetzbaren Befestigungsmitteln, insbesondere Schrauben, befestigbar sind. Form und Grösse eines distalen Endbereichs, welcher eine distalste Stelle der Platte umfasst, sind so ausgebildet, dass der distale Endbereich genau und nur an einem Knochen aus der distalen Reihe der Handwurzelknochen (d.h. Handwurzelknochen aus der Gruppe Os trapezium, Os trapezoideum, Os capitatum, und Os hamatum) anlegbar ist, ohne dabei einen Teil der lateral benachbarten Knochen zu überdecken..

Eine erfindungsgemässe Platte erstreckt sich daher nicht bis zu den Mittelhandknochen. Dass der distale Endbereich genau einem Handwurzelknochen aus der Gruppe Os trapezium, Os trapezoideum, Os capitatum, und Os hamatum anlegbar ist, soll hier nicht so verstanden werden, dass der distale Bereich als Ganzes nicht über einen oder mehrere der weiteren Handwurzelknochen anliegt. Insbesondere kann ein distaler Bereich zusätzlich auch auf einem der Knochen Os scaphoideum, Os lunatum oder Os triquetrum (d.h. der proximalen Reihe der Handwurzelknochen) aufliegen und auf einem dieser Knochen auch verschraubt werden. Allerdings liegt der distale Endbereich nicht auf einem der letztgenannten Knochen und wird auch nicht daran befestigt.

Typischerweise kann die erfindungsgemässe Platte nach einer Entfernung der proximalen Reihe der Handwurzelknochen (Proximal Row Carpectomy) eingesetzt werden. In einem solchen Fall wird die erste, proximale Knochenreihe der Handwurzelknochen (Os scaphoidum, Os lunatum und Os triquetrum) entfernt. In diesem Fall ist die erfindungsgemässe Platte an nur genau einem der distalen Handwurzelknochen und insbesondere am Capitatum angelegt und befestigt.

Mit der erfindungsgemässen Platte wird dann lediglich genau ein Handwurzelknochen aus der Gruppe Os trapezium, Os trapezoideum, Os capitatum, und Os hamatum mit dem Radius versteift. Dadurch kann trotz Versteifung eine relativ grosse Bewegungsfreiheit für den Patienten ermöglicht werden. Falls keine Proximal Row Carpectomy erfolgt, kann die Versteifung mit dem Radius und/oder mit einem Handwurzelknochen der proximalen Reihe erfolgen.

Indem die Form und Grösse des distalen Bereichs erfindungsgemäss ausgewählt wird, ist es ermöglicht, dass die Platte nur an einem der genannten Knochen und insbesondere am Os capitatum anliegt und befestigt wird, ohne dass die Platte an benachbarte Knochen angelegt werden muss. Bevorzugt weist die distalste Stelle eine maximale Breite auf, die etwa der maximalen Breite eines menschlichen Capitatums entspricht.

Benachbarte Handwurzelknochen aus der distalen Knochenreihe, also typischerweise Os hamatum und Os trapezoideum werden von der Platte nicht bedeckt.

Der distale Bereich und insbesondere das distalste Ende hat bevorzugt eine Breite von 10 mm bis 15 mm, vorzugsweise 12 mm bis 14 mm, besonders bevorzugt etwa 13.5 mm. Die Breite des distalen Bereichs wird senkrecht zur Längsachse zwischen den zwei lateralsten Stellen des distalen Bereichs (bzw. deren Projektion parallel zur Längsachse) gemessen. Diese Breiten haben sich als vorteilhaft für eine genügende jedoch nicht zu einschränkende Versteifung herausgestellt. Diese Breite ist bevorzugt auch die grösste Breite der Platte insgesamt.

Der distale Bereich hat bevorzugt eine Länge von 15 mm bis 30 mm, vorzugsweise 15 mm bis 20 mm oder 20 mm bis 27 mm, besonders bevorzugt 16-18 oder 21-24mm. Diese Längen ermöglichen eine Versteifung des Handgelenks, ohne dass sich die Platte in die Mittelhand erstreckt oder zu weit/zu wenig auf dem Radius des Patienten erstreckt. Eine zu kurze Länge kann zu einer unzulänglichen Versteifung führen. Eine zu lange Erstreckung kann sich negativ auf den Tragekomfort der Platte auswirken

Je nach Indikation sind Platten mit unterschiedlichen Längen denkbar. Eine kürzere Platte kann zu der Versorgung nach einer proximal row carpectomy oder bei kleinen Patienten ohne proximal row carpectomy eingesetzt werden, eine längere Platte zur Versorgung unter Beibehalt der proximalen Reihe der Handwurzelknochen oder nach einer proximal row carpectomy bei grossen Patienten. Typischerweise ist die Länge des distalen Bereichs der oben genannten kürzeren Platte im bevorzugten Bereich von 15 mm bis 20 mm im Fall einer proximal row carpectomy bzw. beim kleinen Patienten. Die Länge des distalen Bereichs der längeren Platte beträgt 20 mm bis 27 mm im Fall einer Versorgung unter Beibehaltung der proximalen Knochenreihen bzw. beim grossen Patienten nach einer proximal row carpectomy.

Ein distalstes Plattenloch des proximalen Bereichs kann maximal 20 mm bis 35 mm, vorzugsweise 22 mm bis 26 mm oder 30 mm bis 32 mm, von der distalsten Stelle der Platte beabstandet sein. Hier wird als Abstand vom distalsten Plattenloch des proximalen Bereichs bis zur distalsten Stelle der Platte die Länge einer direkten Strecke von einem Zentrum des Plattenlochs bis zur distalsten Stelle der Platte verstanden.

Auch hier beziehen sich die vorgenannten kürzeren und längeren Abstände auf eine Versorgung unter Entfernung beziehungsweise unter Beibehaltung der proximalen Knochenreihe bzw. bei kleinen oder grossen Patienten.

Vorzugsweise weist die Platte eine Gesamtlänge entlang der Längsachse im Bereich von 55 bis 85 mm, vorzugsweise je nach Art der Versorgung vom 59 mm bis 62 mm bzw. 65 mm bis 68 mm auf.

Dieser Längenbereich hat sich als vorteilhaft herausgestellt, um eine hinreichende Versteifung zwischen Handwurzelknochen und Radius zu erreichen, ohne dass die Bewegungsfreiheit übermässig eingeschränkt wird.

Der proximale Bereich weist vorzugsweise eine Länge von 35 bis 60 mm, vorzugsweise 40mm bis 50 mm, auf. Im proximalen Bereich kann die Platte aber auch länger ausgebildet sein, ohne die bestimmungsgemässe Funktion zu beeinträchtigen.

Vorzugsweise verläuft die Platte in Längsrichtung zumindest in einer ersten Fläche oder mit einer ersten Tangente entlang der Längsachse und in Längsrichtung zusätzlich in einer zur ersten Fläche oder ersten Tangente unter einem Winkel verlaufenden zweiten Fläche oder zweiten Tangente entlang der Längsachse.

Die Platte kann ausserdem in Längsrichtung in einer zur ersten Fläche beabstandeten und dazu etwa parallelen dritten Fläche verlaufen.

Diese Ausführungsform ist insbesondere im Zusammenhang mit der vorstehend beschriebenen längeren Platte für eine Versorgung unter Beibehaltung der proximalen Reihe der Handwurzelknochen bevorzugt.

Die zweite Fläche oder die zweite Tangente weist bevorzugt einen Winkel von etwa 45° zur ersten Fläche auf und/oder verläuft über eine Länge von 7.5 mm bis 8.5 mm, vorzugsweise 8.0 mm. Die Platte verläuft ausserdem in einer bevorzugten Ausführungsform nach der zweiten Fläche in proximaler Richtung in der dritten Fläche, oder der drittem Tangente entlang der Längsachse, insbesondere über eine Länge von 8.0 mm bis 9.0 mm, vorzugsweise 8.5 mm.

Nach der zweiten Fläche oder nach der dritten Fläche kann die Platte in proximaler Richtung in einer vierten Fläche, oder entlang einer vierten Tangente in die erste Fläche übergehen, wobei die vierte Fläche oder die vierte Tangente bevorzugt einen Winkel von etwa 35° zur ersten Fläche aufweist und/oder über eine Länge von 8.5 mm bis 9.5 mm, vorzugsweise 9.3 mm verläuft. Vorliegend wird auf Flächen verwiesen. Die Flächen verlaufen typischerweise je im Wesentlichen in einer Ebene.

Im Fall einer kurzen Platte für eine Versorgung bei einer proximal row carpectomy oder kleinen Patienten erfolgt der Übergang direkt von der zweiten in die vierte Ebene. Im Fall einer Versorgung unter Beibehaltung der proximalen Knochenreihe oder beim grossen Patienten befindet sich zwischen der zweiten und der vierten Ebene die dritte Ebene.

Es versteht sich von selbst, dass die genannten Flächen nicht vollständig planar sein müssen. Eine oder mehrere Krümmungen sind denkbar. Aus diesem Grund wird vorstehend jeweils auf Flächen oder Tangenten verwiesen.

Ein derartiger Verlauf mit mehreren Flächen/Tangenten entlang der Längsrichtung ermöglicht eine gute Anpassung der Platte an die Anatomie der Handwurzelknochen. Insbesondere ermöglicht eine leicht abgerundete Sattelform im distalen Bereich, dass der distale Bereich gut an der Handwurzel aufliegt. Alternativ zur Sattelform ist es auch denkbar, dass zumindest planar verlaufende Abschnitte, welche einen Winkel bilden, in Längsrichtung vorhanden sind. Es sind aber auch vollständig flache Platten denkbar.

Vorzugsweise weist der proximale Bereich der Platte eine maximale Breite von 8 bis 20 mm, vorzugsweise 9 mm bis 15 mm, besonders bevorzugt 13.5, auf. Als Breite wird wiederum eine senkrecht zur Längsachse gemessene Distanz zwischen den zwei lateralsten Stellen des proximalen Bereichs (bzw. deren Projektion parallel zur Längsachse) verstanden.

Vorzugsweise weist der distale Bereich in Draufsicht gesehen nebeneinander (d.h. innerhalb eines Winkelbereichs von +/-45° bezogen auf eine Achse etwa senkrecht zur Längsachse A) je maximal zwei, vorzugsweise genau zwei, Plattenlöcher zur Befestigung der Platte an dem genau einen Handwurzelknochen auf.

Zwei Plattenlöcher, welche in Draufsicht gesehen nebeneinander liegen, ermöglichen eine sichere Befestigung des distalen Endbereichs an einem einzigen Handwurzelknochen.

Vorzugsweise weist der distale Endbereich maximal sechs, vorzugsweise genau sechs, Plattenlöcher zur Aufnahme von Knochenschrauben zur Befestigung der Platte am Handwurzelknochen auf. Selbstverständlich kann die Platte daneben weitere Löcher, z.B. für einen K-Draht, aufweisen. Die distalen vier Plattenlöcher sind für das Capitatum gedacht, die proximalen zwei Plattenlöcher für das Lunatum. Bei einer grossen Hand und einer proximal row carpectomy wird eine kurze Platte verwendet und es werden bis zu sechs Schrauben in das Capitatum geschraubt. Bei einer kleinen Hand ohne proximal row carpectomy wir eine kurze Platte verwendet und Capitatum und Lunatum verschraubt.

Die Plattenlöcher können bevorzugt mit einer dem Fachmann an sich bekannten Verblockungskontur versehen sein, welche die winkelstabile Befestigung von Knochenschrauben ermöglicht. Solche Konturen sind beispielsweise aus WO 2004/086990 A1 bekannt.

Alternativ ist auch eine andere Anzahl Plattenlöcher, wie beispielsweise drei, vier oder fünf Plattenlöcher im distalen Bereich möglich.

Der proximale Bereich weist vorzugsweise zumindest zwei, vorzugsweise drei, vier, fünf, sechs, sieben, acht, neun oder zehn, oder mehr Plattenlöcher in Form von Rund- oder Langlöchern für Knochenschrauben auf. Bevorzugt sind mehrere Rundlöcher und ein Langloch.

Auch im proximalen Bereich können die Plattenlöcher für Knochenschrauben mit Verblockungskonturen versehen sein und/oder es können Löcher für K-Drähte vorgesehen werden.

Dadurch kann der proximale Bereich sicher am Radius befestigt werden.

Es sind auch mehr als zehn Plattenlöcher und/oder mehr oder weniger als ein Plattenloch in Form eines Langlochs im proximalen Bereich denkbar.

Vorzugsweise beinhaltet die Platte Titan oder besteht die Platte aus Titan. Titan(legierungen) sind biokompatibel und widerstandsfähig und haben sich als optimal für Knochenplatten herausgestellt.

Andere biokompatible Materialien, wie andere Metalle/Metalllegierungen oder Kunststoffe sind auch denkbar.

Die Erfindung betrifft zudem ein Verfahren zur Versteifung eines Handgelenks mit einer Handgelenksarthrodeseplatte, vorzugsweise einer vorgängig beschriebenen Platte, wobei ein proximaler Bereich der Platte am Radius und/oder an einem Handwurzelknochen der proximalen Reihe befestigt wird und ein distaler Bereich an genau einem der folgenden Handwurzelknochen befestigt wird: Os trapezium, Os trapezoideum, Os capitatum, und Os hamatum.

Eine Befestigung an genau einem Handwurzelknochen pro Reihe erreicht eine genügende, jedoch nicht zu grossflächige Versteifung.

Insbesondere wird die Platte an keinem Mittelhandknochen befestigt. Zudem wird die Platte nicht an zwei lateral nebeneinander liegenden Handwurzelknochen, sondern lediglich maximal an zwei in Längsrichtung liegenden Handwurzelknochen, befestigt. Vorzugsweise wird der distale Bereich mit maximal sechs Schrauben an dem oder den Handwurzelknochen befestigt.

Vorzugsweise wird der proximale Bereich mit zwei bis sechs Schrauben befestigt.

Weitere Aspekte der Erfindung sind den nachfolgenden Figuren zu entnehmen. Es zeigen schematisch:
- Fig. 1:: Die Anatomie um das Handgelenk;
- Fig. 2:: Eine erfindungsgemässe Handgelenksarthrodeseplatte,
- Figur 3a:: Eine Seitenansicht einer ersten erfindungsgemässen Handgelenksarthrodeseplatte,
- Figur 3b:: Draufsicht auf die Platte gemäss Figur 3a,
- Figur 4a:: Eine Seitenansicht einer alternativen Ausführungsform einer erfindungsgemässen Handgelenksarthrodeseplatte und,
- Figur 4b:: Eine Draufsicht auf die Platte gemäss Figur 4a.

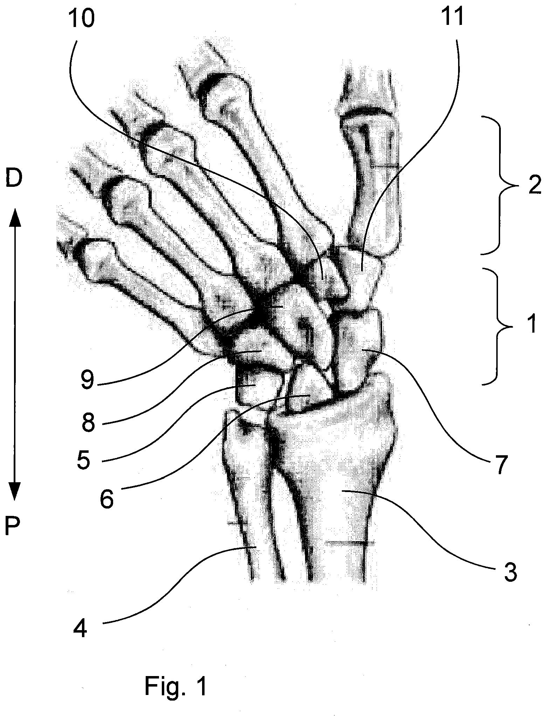

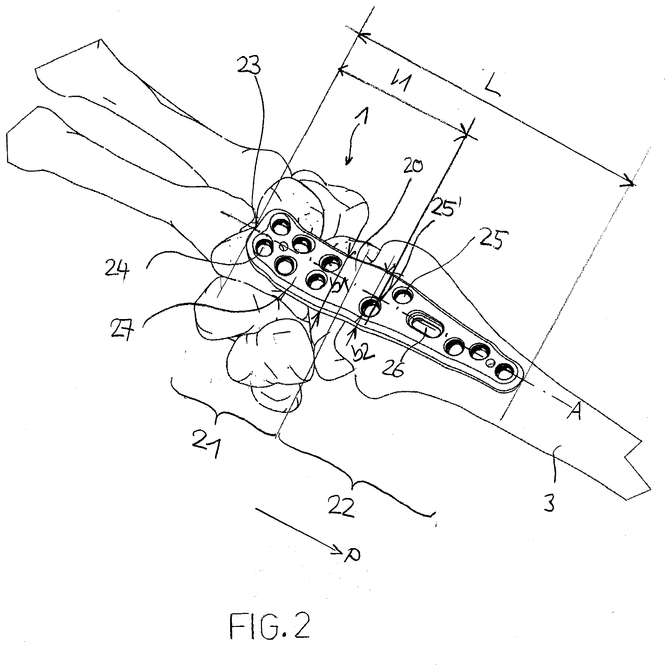

Die Figur 1 zeigt schematisch die Anatomie um das Handgelenk. In distaler Richtung D grenzt die Handwurzel 1 an Radius 3 und Ulna 4 an. Distal D von der Handwurzel 1 liegt die Mittelhand 2. Die Handwurzel 1 weist die Knochen Os triquetrum 5, Os lunatum 6 und Os scaphoideum 7 auf. Distal D von drei Handwurzelknochen 5, 6, 7 liegen befinden sich die Handwurzelknochen Os hamatum 8, Os capitatum 9, Os trapezoideum 10 und Os trapezium 11. Ein distaler Endbereich 29 mit der distalsten Stelle 23 der erfindungsgemässen Handgelenksarthrodeseplatte 20 (siehe Figur 2) ist auf einem einzigen der vier Handwurzelknochen Os hamatum 8, Os capitatum 9, Os trapezoideum 10 und Os trapezium 11 anlegbar. Die Knochenplatte 21 erstreckt sich nicht bis in die Mittelhand 2. Zwischen dem proximalen Bereich 22 und den distalen Stellen 23 kann die Platte über oder an einem der weiteren Knochen Os triquetrum 5, Os lunatum 6 oder Os scaphoideum 7 liegen, ohne aber daran befestigt zu sein.

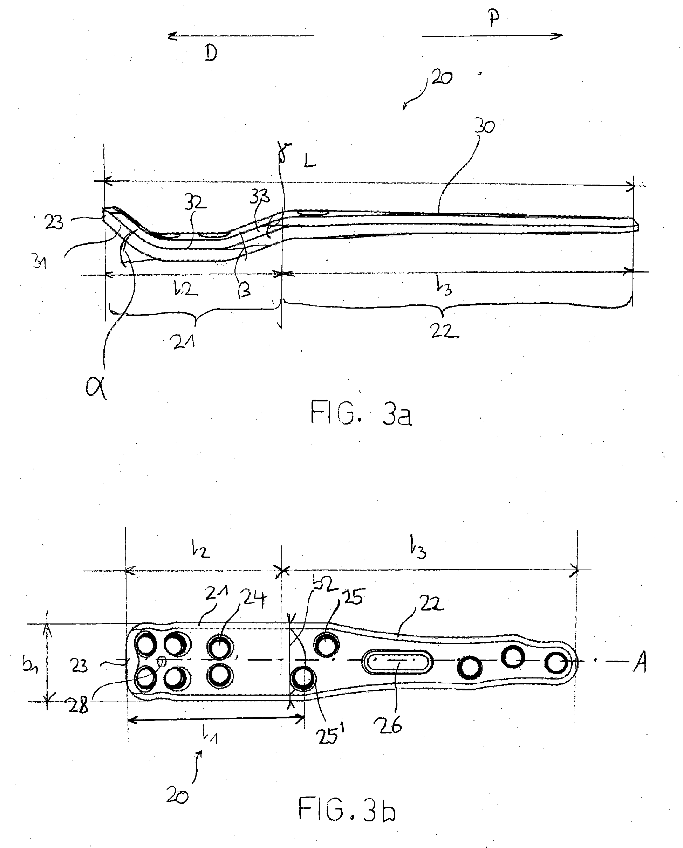

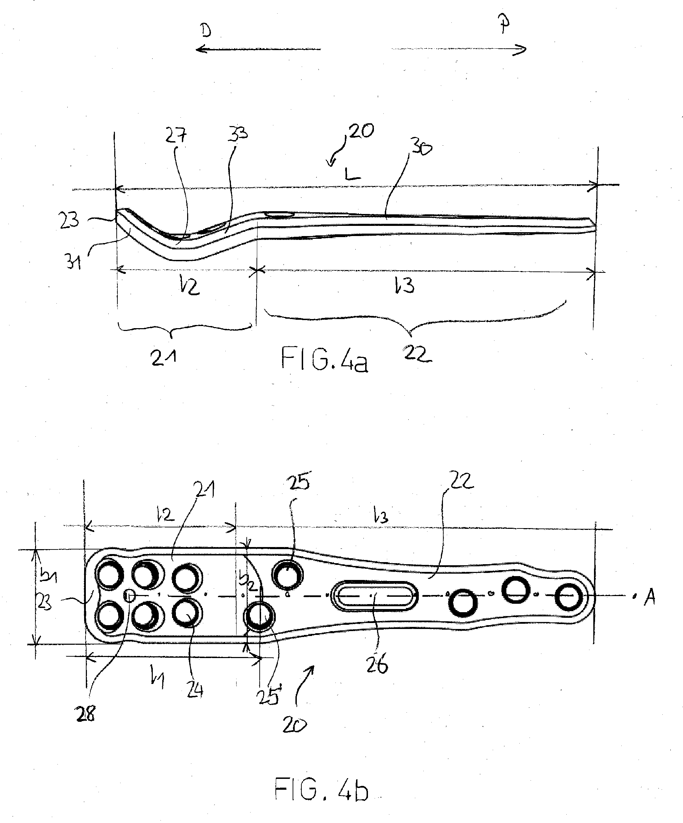

Figur 2 zeigt eine erfindungsgemässe Knochenplatte 20 in bestimmungsgemässer Lage. Die Platte 20 ist aus Reintitan oder einer Titanlegierung gefertigt und weist eine Längsachse A auf. Entlang der Längsachse A weist die Platte eine Länge L von etwa 55 mm bis 85 mm auf (siehe dazu auch Fig. 3a/3b und 4a/4b). auf.

Die Platte 20 besteht aus einem distalen Bereich 21 und einem in proximaler Richtung P angrenzenden proximalen Bereich 22. Der distale Bereich 21 hat entlang der Längsachse A je nach Art der Versorgung eine Länge von 16 mm bis 28 mm. Der proximale Bereich 22 hat entlang der Längsachse A eine Länge von ca. 40 mm bis 50mm.

Der proximale Bereich 22 der Platte 20 liegt auf dem Radius 3 und ist daran befestigbar. Der distale Bereich 21 liegt auf der Handwurzel 1 und ist daran befestigbar. Der distale Endbereich 29 weist sechs Plattenlöcher 24 (aus Gründen der Übersicht nur einmal bezeichnet) auf. Mit Schrauben (nicht gezeigt), ist der distale Endbereich 29 durch die Plattenlöcher 24 an einen einzigen Handwurzelknochen aus der Gruppe Os hamatum 8, Os capitatum 9, Os trapezoideum 10 und Os trapezium 11 (siehe Fig. 1) befestigbar, ohne dass lateral benachbarte Knochen bedeckt werden. Der proximale Bereich 22 weist mehrere Plattenlöcher auf, konkret fünf Rundlöcher 25, 25' (aus Gründen der Übersicht nur teilweise bezeichnet) und ein Langloch 26 zur Befestigung des proximalen Bereichs mit Schrauben (nicht gezeigt) am Radius 3 auf.

Der distale Bereich 21 der Platte erstreckt sich daher über die Handwurzel 1. Der distale Endbereich 29 liegt jedoch nur auf einem der Handwurzelknochen 8, 9, 10, 11 und wird auch nur an diesem einzelnen Handwurzelknochen 8, 9, 10, 11 befestigt. Kein Bereich der Platte wird an der Mittelhand 2 befestigt oder kontaktiert diese.

Der distale Bereich 21 weist eine sattelförmige Vertiefung 27 auf. Aufgrund der Vertiefung 27 verläuft die Platte 20 am distaleren Ende des distalen Endbereichs 29 in einem Winkel von etwa 10° bezüglich eines proximaleren Bereichs des distalen Endbereichs 29. Durch diese Vertiefung 27 wird der Tragekomfort der Platte 20 erhöht.

Die Platte 20 weist im proximalen Bereich 22 die breiteste Stelle mit einer Breite b2 von etwa 13.5 mm auf. Der distale Bereich hat eine maximale Breite b1 von ebenfalls etwa 13.5 mm. Die Breiten b1, b2 bemessen sich auf einer Strecke senkrecht zur Längsachse zwischen den lateralsten Stellen, bzw. deren Projektionen auf einer Achse parallel zur Längsachse A.

Der Abstand 11 vom distalsten Plattenloch 25' des proximalen Bereichs 22 bis zum distalsten Ende 23 ist je nach Art der Versorgung etwa 25 mm (siehe nachfolgende Beschreibung einer kurzen Platte in Figuren 4a und 4b) oder 31 mm (siehe nachfolgende Beschreibung einer langen Platte in Figuren 3a und 3b). Die Länge 11 wurde aus Übersichtsgründen ausserhalb der Platte eingezeichnet. Die Länge 11 bemisst sich von einem Zentrum des distalsten Plattenlochs 25' des proximalen Bereichs 22 auf direktem Weg zur distalsten Stelle 23 der Platte 20.

Figur 3a zeigt eine erste Ausführungsform einer erfindungsgemässen Platte 20 in Seitenansicht. Diese Platte ist für eine Versorgung unter Beibehaltung der proximalen Reihe der Handwurzelknochen oder für grosse Patienten nach einer proximal row carpectomy vorgesehen. Im proximalen Abschnitt 22 mit einer Länge L3 von ca. 43 mm verläuft die Platte in einer ersten Fläche 30.

Vom distalsten Ende 23 her in proximaler Richtung P betrachtet verläuft die Platte 20 im distalen Bereich 21 zuerst in einer bezogen auf die Fläche 30 unter einem Winkel α verlaufenden zweiten Fläche 31. Anschliessend verläuft die Platte immer noch im distalen Bereich in einer dritten Fläche 32, welche etwa parallel zur ersten Fläche 30 verläuft. Über eine vierte Fläche 33 geht die Platte von der dritten Fläche 32 in die erste Fläche 30 über. Der Knick zwischen der vierten Fläche 33 und der ersten Fläche 30 definiert typischerweise auch die Abgrenzung zwischen dem proximalen Bereich 22, welcher auf dem Radius anlegbar ist und dem distalen Bereich 21. Im gezeigten Ausführungsbeispiel beträgt die Länge L2 des distalen Abschnitts 21 18-22 mm.

In Figur 3a betragen die Winkel α zwischen der zweiten Fläche 31 und der dritten Fläche 32 etwa 45° und der Winkel β zwischen der dritten Fläche 32 und der vierten Fläche 33 sowie der Winkel γ zwischen der vierten Fläche 33 und der ersten Fläche 30 etwa 35°.

Im konkreten Beispiel sind die erste Fläche 30 und die dritte Fläche 32 in Figur 3a etwa parallel zueinander. Es ist aber auch denkbar, einen kleinen Winkel zwischen erster Fläche 30 und dritter Fläche 32 vorzusehen, beispielsweise einen Winkel von bis zu 10°.

Figur 3b zeigt eine Draufsicht auf die Platte aus Figur 3a. Gleiche Bezugszeichen bezeichnen gleiche Teile. In Figur 3b sind zusätzlich die verschiedenen Löcher, insbesondere Plattenlöcher 24 (im distalen Bereich 21) und 25, 25' (im proximalen Bereich 22) zur Aufnahme von Knochenschrauben, ein Plattenloch in Form eines Langlochs 26 sowie ein Loch 28 zur Aufnahme eines K-Drahts gezeigt. Im proximalen Bereich weist die Platte eine maximale Breite b2 von etwa 13.5 mm auf und verjüngt sich in proximaler Dichtung P. Im distalen Bereich 21 weist die Platte eine im wesentlichen konstante Breite b1 von ebenfalls etwa 13,5 mm auf.

Die Figuren 4a und 4b zeigen analog zu den Figuren 3a und 3b eine alternative Ausführungsform einer erfindungsgemässen Platte, welche für eine Versorgung einer normal grossen Hand nach einer proximal row carpectomy oder auch einer kleinen Hand ohne diesen Eingriff geeignet ist. Entsprechend fehlt die dritte Fläche 32, welche in Figur 3a sichtbar ist: Stattdessen geht die erfindungsgemässe Platte gemäss Figuren 4a und 4b unmittelbar von der zweiten Fläche 31 in die vierte Fläche 33 über. Entsprechend ist die Länge L2 des distalen Abschnitts 21 gemäss Figur 4a kürzer als gemäss Figur 3a und beträgt typischerweise 17mm. Die Länge L3 des proximalen Abschnitts 22 ist hingegen in beiden Ausführungsformen etwa gleich. Im Ausführungsbeispiel gemäss Figur 4a geht die zweite Fläche 31 in einem leichten Radius in die vierte Fläche 33 über. Die Winkellage der zweiten Fläche 31 und der dritten Fläche 33 zueinander und gegenüber der ersten Fläche 30 entsprechen ungefähr den Winkellagen gemäss dem Ausführungsbeispiel von Figur 3a.
Der Abstand des distalsten Plattenlochs 25' des proximalen Teils 22 (in Figuren 3b und 4b mit 11 bezeichnet) vom distalsten Ende 23 beträgt typischerweise 25 mm (Figur 3a) oder 17 mm (Figur 4a) .

Die Längen der zweiten, dritten und vierten Flächen betragen typischerweise zwischen 7.5 mm und 10.0 mm. Im konkreten Ausführungsbeispiel beträgt die Länge der zweiten Fläche etwa 8.0 mm, die Länge der dritten Fläche etwa 8.7 mm und die Länge der vierten Fläche etwa 9.3 mm.

## Patentansprüche

1. Handgelenksarthrodeseplatte (20) bestehend aus einem distalen (21) und einem proximalen (22) Bereich, welcher in Richtung einer Längsachse (A) an den distalen Bereich (21) anschliesst, wobei Form und Grösse der Platte (20) so ausgebildet sind, dass der distale Bereich (21) der Platte an der Handwurzel (1) und der proximale Bereich (22) der Platte am Radius (3) mit Hilfe von in Plattenlöchern einsetzbaren Befestigungsmitteln, insbesondere Schrauben, befestigbar sind, **dadurch gekennzeichnet, dass** Form und Grösse eines distalen Endbereichs (29), welcher eine distalste Stelle (23) der Platte (20) umfasst, so ausgebildet sind, dass der distale Endbereich (29) genau und nur an einem Handwurzelknochen aus der Gruppe Os trapezium (11), Os trapezoideum (10), Os capitatum (9), und Os hamatum (8) anlegbar ist, ohne dabei einen Teil der lateral benachbarten Knochen zu überdecken.

2. Platte (20) nach Anspruch 1, wobei die distalste Stelle (23) eine maximale Breite (b1) aufweist, die etwa der maximalen Breite eines menschlichen Capitatums entspricht.

3. Platte (20) nach einem der der vorhergehenden Ansprüche, wobei der distale Bereich (21) und insbesondere das distalste Ende (23) eine Breite (b1) von 10 mm bis 15 mm, bevorzugt 12 mm bis 14 mm, vorzugsweise 13.5 mm aufweist.

4. Platte (20) nach einem der vorhergehenden Ansprüche, wobei der distale Bereich (21) eine Länge (12) von 15 mm bis 30 mm, vorzugsweise 15 mm bis 20 mm oder 20 mm bis 27 mm, aufweist.

5. Platte (20) nach einem der vorhergehenden Ansprüche, wobei ein distalstes Plattenloch (24') des proximalen Bereichs (22) einen Anstand (11) von maximal 20 mm bis 35 mm, vorzugsweise 22 mm bis 26 mm oder 30 mm bis 32 mm, von der distalsten Stelle (23) der Platte aufweist.

6. Platte (20) nach einem der vorhergehenden Ansprüche, wobei die Platte eine Länge (L) entlang der Längsachse (A) im Bereich von 55 bis 85 mm, vorzugsweise 59 mm bis 62 mm oder 65 mm bis 68 mm aufweist.

7. Platte (20) nach einem der vorhergehenden Ansprüche, wobei der proximale Bereich (22) eine Länge(13) von 35 bis 60 mmm, vorzugsweise 40mm bis 50 mm, aufweist.

8. Platte (20) nach einem der vorhergehenden Ansprüche, wobei die Platte (20) in Längsrichtung zumindest in einer ersten Fläche (30) oder mit einer ersten Tangente entlang der Längsachse (A) verläuft und in einer zur ersten Fläche (30) oder ersten Tangente unter einem Winkel (α)verlaufenden zweiten Fläche (31) oder zweiten Tangente entlang der Längsachse (A) verläuft.

9. Platte (20) nach einem der vorhergehenden Ansprüche, wobei die Platte (20) in Längsrichtung in einer zur ersten Fläche (30) beabstandeten und dazu im Wesentlichen parallelen dritten Fläche (32) verläuft.

10. Platte (20) nach einem der Ansprüche 8 oder 9, wobei der Winkel (α) zwischen der zweiten Fläche (31) oder der zweiten Tangente und der ersten Fläche (30) oder ersten Tangente etwa 45° beträgt und/oder die zweite Fläche (31) über eine Länge von 7.5 mm bis 8.5 mm, vorzugsweise 8.0 mm verläuft.

11. Platte (20) nach Anspruch 10, wobei die Platte (20) nach der zweiten Fläche (31) in proximaler Richtung (P) in der dritten Fläche (32) oder der dritten Tangente entlang der Längsachse (A) verläuft, insbesondere über eine Länge von 8.0 mm bis 9.0 mm, vorzugsweise 8.5 mm.

12. Platte nach Anspruch 10 oder 11, wobei die Platte (20) nach der zweiten Fläche (31) oder nach der dritten Fläche (32) in proximaler Richtung (P) in einer vierten Fläche (33) oder entlang einer vierten Tangente in die erste Fläche (30) übergeht, wobei die vierte Fläche (33) oder die vierte Tangente bevorzugt einen Winkel (γ) von etwa 35° zur ersten Fläche aufweist und/oder über eine Länge von 8.5 mm bis 9.5 mm, vorzugsweise 9.3 mm verläuft.

13. Platte (20) nach einem der vorhergehenden Ansprüche, wobei der proximale Bereich (22) der Platte (20) eine maximale Breite (b2) von 8 bis 20 mm, vorzugsweise 9 mm bis 15 mm besonders bevorzugt 13.5 aufweist.

14. Platte (20) nach einem der vorhergehenden Ansprüche, wobei der distale Bereich (21) in einem Winkelbereich von +/- 45° zu einer Senkrechten zur Längsachse (A) maximal vier, vorzugsweise genau zwei, Plattenlöcher (24) zur Befestigung der Platte (20) an dem genau einen Handwurzelknochen (8, 9, 10, 11) aufweist.

15. Platte (20) nach einem der vorhergehenden Ansprüche, wobei der distale Endbereich (29) maximal sechs, vorzugsweise genau sechs, Plattenlöcher (24) zur Aufnahme einer Knochenschraube zur Befestigung der Platte (20) an dem genau einen Handwurzelknochen (8, 9, 10, 11) aufweist.

16. Platte (20) nach einem der vorhergehenden Ansprüche, wobei die Platte (20) Titan beinhaltet oder aus Titan besteht.

17. Verfahren mit einer Handgelenksarthrodeseplatte (20), vorzugsweise einer Platte (20) nach einem der Ansprüche 1 bis 12, wobei ein proximaler Bereich (22) der Platte am Radius (3) und/oder an einem Handwurzelknochen der proximalen Reihe befestigt wird und ein distaler Bereich (21) der Platte (20) an genau einem Handwurzelknochen (8, 9, 10, 11) aus der Gruppe Os trapezium (11), Os trapezoideum (10), Os capitatum (9), und Os hamatum (8) befestigt wird.

18. Verfahren nach Anspruch 14, wobei der distale Bereich (21) mit maximal vier Schrauben an dem Handwurzelknochen (8, 9, 10, 11) befestigt wird.
